(19)

Europäisches
Patentamt
European
Patent Office
Office européen
des brevets

(11) **EP 2 397 978 A1**

(12) **EUROPEAN PATENT APPLICATION**

(43) Date of publication:
  **21.12.2011 Bulletin 2011/51**

(51) Int Cl.:
  *G06Q 10/00* (2006.01)    *G06Q 50/00* (2006.01)

(21) Application number: **11158611.1**

(22) Date of filing: **17.03.2011**

(84) Designated Contracting States:
  **AL AT BE BG CH CY CZ DE DK EE ES FI FR GB GR HR HU IE IS IT LI LT LU LV MC MK MT NL NO PL PT RO RS SE SI SK SM TR**
  Designated Extension States:
  **BA ME**

(30) Priority: **15.06.2010 KR 20100056769**

(71) Applicant: **Genome Research Foundation Gyeonggi-do 443-270 (KR)**

(72) Inventors:
  • **Ghang, Ho**
    **Gyeonggi-do 446-904 (KR)**
  • **Bhak, Jong**
    **Gyeongsangnam-do 668-831 (KR)**
  • **Kim, Byung Chul**
    **Gyeonggi-do 443-470 (KR)**
  • **Lee, Sung Hoon**
    **Daejeon 305-333 (KR)**
  • **Kim, Tae Hyung**
    **Gyeonggi-do 446-914 (KR)**
  • **Park, Jinuk**
    **Daejeon 302-120 (KR)**

(74) Representative: **Franke, Dirk**
  **Hards & Franke**
  **Patentanwälte Partnerschaft**
  **Widenmayerstraße 25**
  **80538 München (DE)**

(54) **System and method for forming an online social network using genome information**

(57)    The present invention relates to a system and method for forming an online social network using genome information. To be more specific, an embodiment of the invention may include a web server that creates a database of detailed information of members, including genome information of the member, as inputted from member terminals, and which generates a social network on the world wide web based on the genome information of the members. An embodiment of the invention can be utilized to satisfy the various personal demands of members by analyzing the gene information of members to form a database of the members' genome information and forming a social network online according to the degree of genetic similarity or distance of the genome information.

Fig. 1

EP 2 397 978 A1

## Description

BACKGROUND

1. Technical Field

The present invention relates to a system for forming a social network online using genome information, more particularly to a system and method for forming an online social network using genome information that can satisfy the personal preferences of members by analyzing the genetic information of members to form a database of the members' genome information and forming a social network online according to the degree of genetic similarity or distance of the genome information.

2. Description of the Related Art

The present invention relates to a system and method for forming a social network online using genome information.

A common feature of all living things, including humans, is to reproduce other organisms that have similar traits as the parent. This is referred to as "heredity", and is made possible by the genetic material known as "DNA", which is included in the nucleus of a cell.

The human race has survived and evolved using this phenomenon of heredity, and moreover, all of the biological and sociological activities and development of each individual, including birth, growth, sickness, death, etc., are closely related to the expression of one's genes, as the factors related to such biological and sociological phenomena are stored as encoded genetic information in various genes.

A gene includes the genetic information required for creating a protein, arguably the most important component in maintaining life, and is expressed by the base sequences of DNA, i.e. the sequences formed by the bases, adenine (A), cytosine (C), guanine (G), and thymine (T). A sequence of three consecutive bases within a DNA is referred to as a codon, where a series of codons can be transcribed to an RNA and translated into a particular amino acid. Several amino acids may join together to form proteins, which may be used in forming major structures within the cell, as well as in functioning as hormones, enzymes, etc., to mediate various reactions that determine the fate of a human being.

The term "genome", which is a portmanteau of the words "gene" and "chromosome", refers to the set of all genetic information (DNA) carried by a living organism. The genome information contains information that will determine the phenotype of an organism within the gravitational field and the given space-time of earth for all living things. The greater the similarity between more the genome information of two organisms, the higher the likelihood that the organisms are similar phenotypically.

At present, the genome of a human being is known to consist of about 3 billion pairs of DNA base sequences, and according to the genome project, the number of genes that express a particular function is about 30 to 40 thousand.

Even those genes that express the same characteristic (e.g. race, blood type, etc.) can include different base sequences for each person, and such distinctive gene fragments of an organism are called alleles. The reason why the same gene can have different genetic elements for each organism and thus create differences within individuals is explained by the concept of single-nucleotide polymorphism, or SNP for short. The concept of SNP is of utmost importance in identifying and distinguishing individuals, and the genetic and biological properties of each individual can be determined by the variation of SNP.

Such genetic elements are also related to the diseases suffered by people, as the resistance and sensitivity of an individual to a disease, and the severity of the disease suffered by the individual, may vary according to the variation of SNP.

Due to advances in molecular genetics technology, it is currently possible to analyze an individual's genome information quickly and inexpensively. The genome information can be utilized to recognize beforehand the biological and genetic problems, etc., of each individual, including those related to the personality, sickness, growth, etc., and hence enable their prevention, treatment, or remedy.

Meanwhile, recent advances in the Internet have caused a great increase in people's interest towards social network services (SNS) that are based on the relationships between a person, the person's friends, and the friends' friends, to show the network of personal contacts resulting therefrom.

A typical example in Korea is Cyworld (www.cyworld.com), which provides a simple personal network service that helps establish a personal network between members based on the *ilchon* relations formed by the members; another example is MySpace (www.myspace.com), which provides a social network service to numerous members worldwide.

In these conventional social network services, a person may form a personal network based on a personally created list of acquaintances (a friend list) in the service which the person has joined.

In other words, the server of the social network service may receive information on a first user and the contacts of the first user from the first user's computer and may receive information on a second user and the contacts of the second user from the second user's computer. Afterwards, when the server receives contact search conditions from the first user's computer, if the information of the second user corresponds to the contact search conditions and there are common contacts between the first user and the second user, then a summary of the second user may be transmitted to the first user, to form a social network.

However, this conventional method of forming a social network provides a simple form of personal networks using only general information of the members, such as location, alumni, hobbies, preferences, etc.

Furthermore, with the conventional method of forming a social network, it is difficult to recognize the genetic relationships between members of the social network.

SUMMARY

An aspect of the invention is to provide a system and method for forming a social network online using genome information that can satisfy the various personal demands of members by analyzing the gene information of members to form a database of the members' genome information and forming a social network online according to the degree of genetic similarity or distance of the genome information.

Another aspect of the invention is to provide a system and method for forming a social network online using genome information that can form a social network between members having genetic similarity, thus enabling members to use the network immediately when needed (for example, when in need of a transplant, etc.).

To achieve the objectives above, an aspect of the invention provides a system for forming an online social network using genome information that includes a web server, which creates a database of detailed information of members, including genome information of the member, as inputted from member terminals, and which generates a social network on the world wide web based on the genome information of the members.

Here, the web server can include: a membership signup unit, to which a membership signup request may be inputted from a member terminal; a member information input unit, to which the detailed information of a member, including the genome information of the member, may be inputted from the member terminal from which the membership signup request is inputted; a member information database, in which the detailed information of the members may be stored; and a social network generator unit, which may perform the processing necessary to form a social network on the world wide web by way of a particular group, based on the genome information of members stored in the member information database, when a request for generating a social network is inputted from a member terminal.

The social network generator unit can include: a group generation processing unit, which may ask whether to generate a group of members having high genetic similarity to a member or a group of members having high genetic distance to the member and generate a group based on the response, when a group generation request for generating a group is inputted from a member terminal; and a group signup processing unit, which may ask whether to join a group of members having high genetic similarity to a member or a group of members having high genetic distance to the member and perform the processing necessary to join the member to a group based on the response, when a request for joining a pre-generated group is inputted from a member terminal.

Upon receiving a response from a member terminal for generating a group of members having high genetic similarity to the member, the group generation processing unit may access the genome information of members stored in the member information database, extract members having high genetic similarity to the genome information of the member, transmit a request message for inviting signup to a group to terminals of the extracted members, and perform the processing necessary to generate a particular group by grouping the member wishing to generate a group with members accepting the request message.

Conversely, upon receiving a response from a member terminal for generating a group of members having high genetic distance to the member, the group generation processing unit may access the genome information of members stored in the member information database, extract members having high genetic distance to the genome information of the member, transmit a request message for inviting signup to a group to terminals of the extracted members, and perform the processing necessary to generate a particular group by grouping the member wishing to generate a group with members accepting the request message.

The group signup processing unit, upon receiving a response from a member terminal for joining a group of members having high genetic similarity to the member, may search for groups formed by members having high genetic similarity to the member, transmit a signup request message for requesting signup to terminals of administrators of the searched groups, and may perform the processing necessary to join the member to the group if group signup authorization is received from a group administrator terminal.

Also, the group signup processing unit, upon receiving a response from a member terminal for joining a group of members having high genetic distance to the member, may search for groups formed by members having high genetic distance to the member, transmit a signup request message for requesting signup to terminals of administrators of the searched groups, and may perform the processing necessary to join the member to the group if group signup authorization is received from a group administrator terminal.

Yet another aspect of the invention provides a method for forming an online social network using genome information that includes: (A) receiving by a web server of detailed information of a member, including genome information, as input from a member terminal; (B) storing by the web server of the detailed information of the member inputted in step (A) in a member information database; (C) receiving by the web server of a request for generating a social network on the world wide web as input from a member terminal; and (D) forming by the web server of a social network on the world wide web by way of a particular group, based on the genome information of members stored in the member information database.

Here, step (C) can include: (C1) receiving by the web server of a group generation request for generating a group as input from the member terminal; and (C2) asking by the web server of whether to generate a group of members having high genetic similarity to the member or a group of members having high genetic distance to the member.

In this case, step (D) can include: (D1) accessing by the web server of the genome information of members stored in the member information database, if as a result of step (C2) a response is received from the member terminal for generating a group of members having high genetic similarity to the member; (D2) extracting by the web server of members having high genetic similarity to the genome information of the member and transmitting a request message for inviting signup to a group to terminals of the extracted members; and (D3) generating by the web server of a particular group by grouping the member wishing to generate a group with members accepting the request message of step (D2).

Also, step (D) can include: (D1') accessing by the web server of the genome information of members stored in the member information database, if as a result of step (C2) a response is received from the member terminal for generating a group of members having high genetic distance to the member; (D2') extracting by the web server of members having high genetic distance to the genome information of the member and transmitting a request message for inviting signup to a group to terminals of the extracted members; and (D3') generating by the web server of a particular group by grouping the member wishing to generate a group with members accepting the request message of step (D2').

Step (C) can include: (C1) receiving by the web server of a request for joining a pre-generated group as input from the member terminal; and (C2) asking by the web server of whether to join a group of members having high genetic similarity to the member or a group of members having high genetic distance to the member.

Here, step (D) can include: (D4) searching by the web server for groups formed by members having high genetic similarity to the member, if as a result of step (C4) a response is received from the member terminal for joining a group formed by members having high genetic similarity to the member; (D5) transmitting by the web server of a signup request message for requesting signup to terminals of administrators of the groups searched in step (D4); and (D6) joining by the web server of the member to a group, if group signup authorization is received from a group administrator terminal.

Step (D) can also include: (D4') searching by the web server for groups formed by members having high genetic distance to the member, if as a result of step (C4) a response is received from the member terminal for joining a group formed by members having high genetic distance to the member; (D5') transmitting by the web server of a signup request message for requesting signup to terminals of administrators of the groups searched in step (D4'); and (D6') joining by the web server of the member to a group, if group signup authorization is received from a group administrator terminal.

Additional aspects and advantages of the present invention will be set forth in part in the description which follows, and in part will be obvious from the description, or may be learned by practice of the invention.

BRIEF DESCRIPTION OF THE DRAWINGS

Figure 1 is a diagram illustrating the overall composition of a system for forming an online social network using genome information according to a preferred embodiment of the invention.
Figure 2 is a block diagram illustrating the internal composition of the web server in Figure 1.
Figure 3 is a flowchart illustrating the operation of a system for forming an online social network using genome information according to a preferred embodiment of the invention.
Figure 4 is a flowchart illustrating the operation of a system for forming an online social network using genome information according to another embodiment of the invention.
Figure 5 is a diagram illustrating a process by which a member is determined to have high genetic similarity or high genetic distance according to a preferred embodiment of the invention.

DETAILED DESCRIPTION

A system for forming an online social network using genome information according to a preferred embodiment of the invention is described as follows with reference to the accompanying drawings.

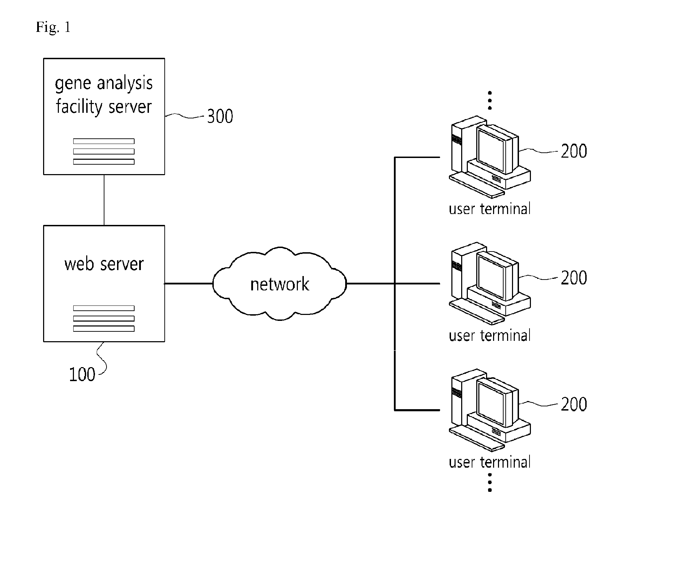

Figure 1 is a diagram illustrating the overall composition of a system for forming an online social network using genome information according to a preferred embodiment of the invention.

Referring to the drawings, an embodiment of the invention may include a web server 100, one or more user terminals 200 (hereinafter referred to as "member terminals"), and a gene analysis facility server 300.

The web server 100 may receive detailed information of the members (e.g. the member's name, address, genome information, hobbies, location, alumni information, whether or not to open the information to the community, etc.), including the genome information of the members, from the member terminals 200 as input and may create a database of the detailed information. The web server 100 may form a social network by generating certain groups on the world wide web based on the genome information of the members. The detailed composition of the web server 100 will be described below in further detail with reference to the drawings.

A member terminal 200 may be a terminal capable of accessing a home page operated by the web server 100 and can be a PC (personal computer), PDA (personal digital assistant), cellular phone, handheld PC, etc.

The gene analysis facility server 300 may, when there is a request from the web server 100, provide the web server 100 with the genome information of a member. Here, it may be preferable to have the gene analysis facility server 300 provide a member's genome information only after performing an authentication procedure and obtaining authentication.

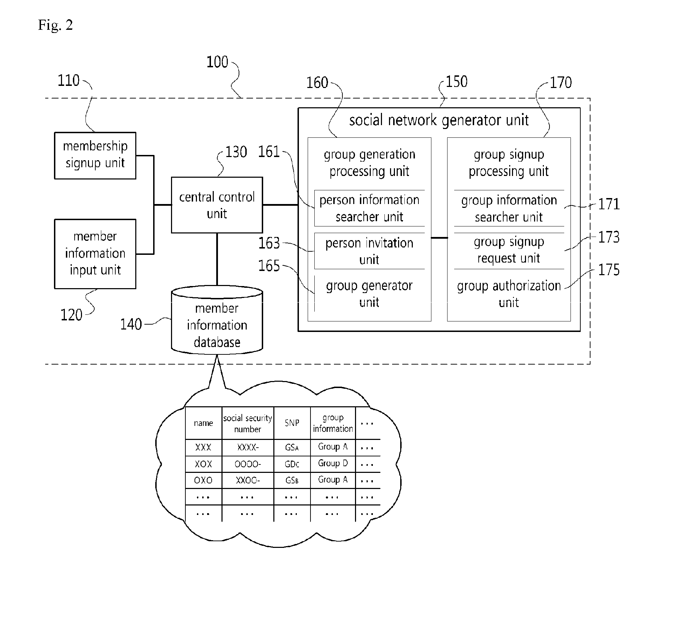

Figure 2 is a block diagram illustrating the internal composition of the web server in Figure 1.

Referring to Figure 2, the web server 100 may include a membership signup unit 110, a member information input unit 120, a central control unit 130, a member information database 140, and a social network generator unit 150.

The membership signup unit 110 may receive membership signup requests as input from the member terminals 200.

The member information input unit 120 may receive the detailed information of the members (e.g. name, address, genome information, hobbies, location, alumni information, whether or not to open the information to the community, etc.), including genome information, as input from the member terminals 200 requesting membership.

The central control unit 130 may perform the processing necessary to store the members' detailed information inputted through the member information input unit 120 in the member information database 140. Also, the central control unit 130 may restrict the service content according to the information received from the member terminal 200 regarding whether or not to open the detailed information of the member to the community (for example, to open the detailed information of the member to other members, open only to the administrator, etc.). In a more specific example, the central control unit 130 may perform the processing necessary to open the information of other members to a member terminal 200 that opts to open its information to other members and conceal the information of other members to a member terminal 200 that opts to conceal its information to other members.

The member information database 140 may store the detailed information of the members. Here, the member information database 140 may preferably store the genome information in the form of a table. Various storage media can be provided for the member information database 140, such as EPROM, flash memory, and external memory, according to the capacity of data stored.

If a request for generating a social network is inputted from a member terminal 200, the social network generator unit 150 may perform the processing necessary to generate a particular group on the world wide web and thereby form an online social network, based on the members' genome information stored in the member information database 140. Here, an online social network refers to a social network formed in an online environment with nodes formed between members by way of the specific interactions between the members. The social network generator unit 150 may include a group generation processing unit 160 and a group signup processing unit 170.

If a group generation request for generating a group is inputted from a member terminal, the group generation processing unit 160 may ask whether to generate a group of members having high genetic similarity or high genetic distance to the member, and may generate a group based on the response. Here, to state that there is high genetic similarity may mean that the genetic similarity is greater than a first threshold value, and to state that there is high genetic distance may mean that the genetic distance is greater than a second threshold value. The group generation processing unit 160 may include a person information searcher unit 161, a person invitation unit 163, and a group generator unit 165.

If a response is received from the member terminal 200 that a group of members having high genetic similarity to the member is to be formed, the person information searcher unit 161 may access the members' genome information of stored in the member information database 140 and extract members that have high genetic similarity compared with the genome information of the member. Here, when searching for members having high genetic similarity, the person information searcher unit 161 may search for members having high similarity using a pre-designated equation such as Equation 1.

[Equation 1]

$$GS_A = \sum P_{AN} / n$$

In the above Equation 1, $GS_A$ represents a query sample Q's group similarity, $P_{AN}$ represents the proportion of shared alleles between individuals, and n represents the number of members of an online social group.

Conversely, if a response is received from the member terminal 200 that a group of members having high genetic distance to the member is to be formed, the person information searcher unit 161 may access the members' genome information of stored in the member information database 140 and extract members that have high genetic distance compared with the genome information of the member. Here, when searching for members having high genetic distance, the person information searcher unit 161 may search for members having high distance using a pre-designated equation such as Equation 2.

[Equation 2]

$$GD_A = \sum Q_{AN} / n$$

In the above Equation 2, $GD_A$ represents a query sample Q's group distance, $Q_{AN}$ represents a Q's individual distance, and n represents the number of members of an on-line social group.

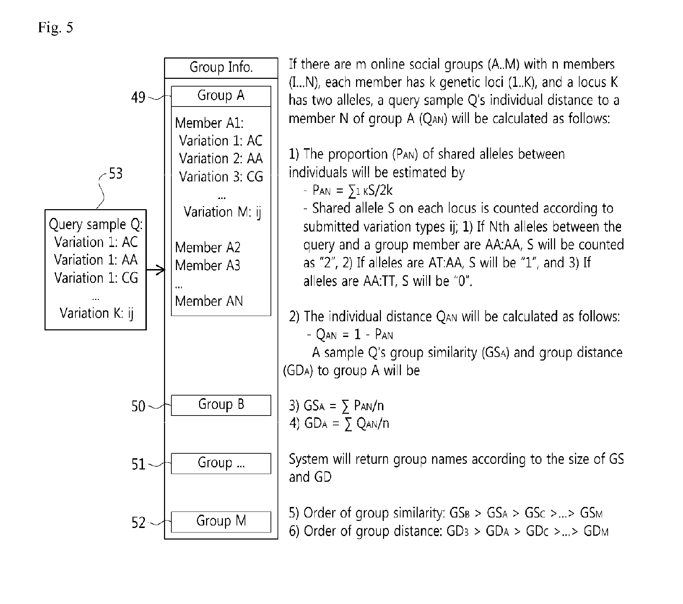

A method for finding a member having high genetic similarity and for finding a member having high genetic distance is described as follows with reference to Figure 5. Figure 5 is a diagram illustrating a process by which a member is determined to have high genetic similarity or high genetic distance according to a preferred embodiment of the invention. The process for finding a member having high genetic similarity or high genetic distance is not limited to the example illustrated in Figure 5, and it is to be appreciated that those of ordinary skill in the art to which the invention pertains can modify an embodiment of the invention in various ways without departing from the spirit and scope of the invention. The method of finding members having high genetic similarity and finding members having high genetic distance is already publicly known in the related art and thus will not be described here in great detail.

The person invitation unit 163 may transmit a request message, which invites signup to a group, to the terminals of the members extracted by the person information searcher unit. Here, the person invitation unit 163 may preferably provide the genome information of the member wishing to create a group together with the request message.

The group generator unit 165 may perform the processing necessary to generate a particular group by grouping the member wishing to generate a group with members accepting the request message.

The group signup processing unit 170, upon receiving a request for joining a pre-generated group inputted from a member terminal 200, may ask whether to join a group of members having high genetic similarity or high genetic distance to the member, and may perform the processing necessary to join the member to a group based on the response. The group signup processing unit 170 may include a group information searcher unit 171, a group signup request unit 173, and a group authorization unit 175.

If a response is received from the member terminal 200 for joining a group formed by members having high genetic similarity, the group information searcher unit 171 may search for groups formed by members having high genetic similarity.

Conversely, if a response is received from the member terminal 200 for joining a group formed by members having high genetic distance, the group information searcher unit 171 may search for groups formed by members having high genetic distance.

The group signup request unit 173 may transmit a signup request message, which requests signup, to the terminal of an administrator of a group searched by the group information searcher unit 171. Here, the group signup request unit 173 may preferably provide the genome information of the member requesting signup together with the signup request message.

If group signup authorization is received from the group administrator's terminal, the group authorization unit 175 may perform the processing necessary to join the member to the group.

The operation of a system for forming an online social network using genome information is described as follows in more detail, with reference to the accompanying drawings.

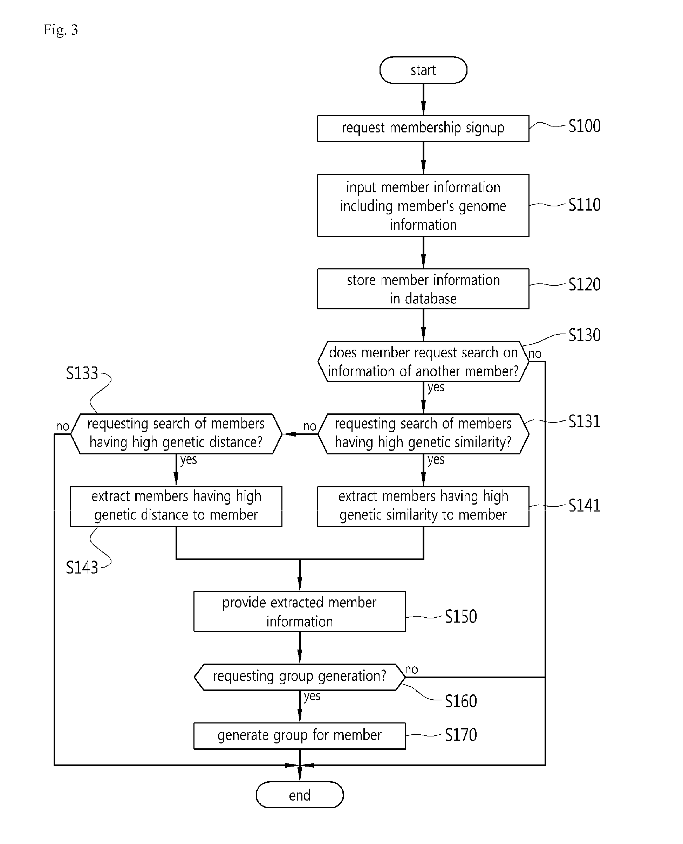

Figure 3 is a flowchart illustrating the operation of a system for forming an online social network using genome information according to a preferred embodiment of the invention.

First, when the web server 100 is inputted with a membership signup request from a member terminal (step S100), the web server 100 may receive the detailed information of the member, including genome information, as input from the member terminal (step S 110).

Then, the web server 100 may store the member's detailed information inputted in step S 110 in the member information database 140 (step S120).

The web server 100 can be inputted with a request for generating a group, in order to form a social network, from a member terminal 200 (step S130).

Then, the web server 100 may ask the member terminal 200 whether to generate a group having members of high genetic similarity to the member (step S131) or generate a group having members of high genetic distance to the member (step S133).

If, as a response to the inquiry, a response for generating a group having members of high genetic similarity to the member is received from the member terminal 200, the web server 100 may access the members' genome information stored in the member information database 140. Then, the web server 100 may extract those members having high genetic similarity as compared with the genome information of the member (step S141).

However, if, as a response to the inquiry, a response for generating a group having members of high genetic distance to the member is received from the member terminal 200, the web server 100 may access the members' genome information stored in the member information database 140. The web server 100 may then extract those members having high genetic distance as compared with the genome information of the member (step S 143).

Afterwards, the web server 100 may provide the member terminal 200 with the information of the extracted members (step S150). Here, the web server 100 may preferably perform the processing such that the information of other members is available only if the member terminal 200 has agreed to open the detailed information of its member to the community.

Afterwards, if there is a request from the member terminal 200, a request message inviting signup to the group may be transmitted to the terminals of the extracted members (step S160). Here, the web server 100 may preferably provide the genome information of the member wishing to create the group together with the request message.

Afterwards, the web server 100 may perform the processing necessary to generate a particular group, and thus form a social network, by grouping the member wishing to generate a group with members accepting the request message.

According to an embodiment of the invention as set forth above, a social network can be formed on the world wide web according to the degree of genetic similarity or distance of the genome information.

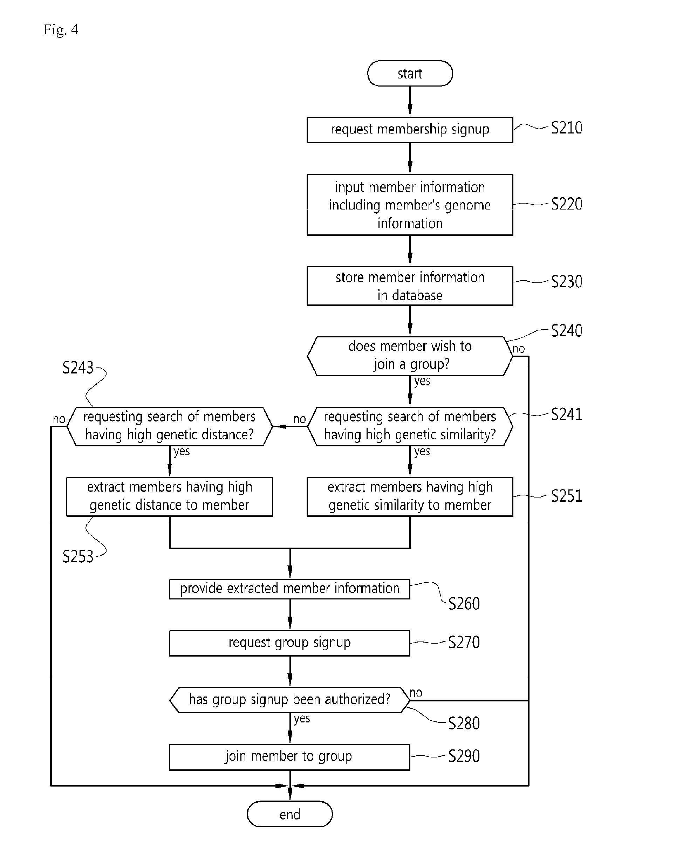

Figure 4 is a flowchart illustrating the operation of a system for forming an online social network using genome information according to another embodiment of the invention.

First, when the web server 100 is inputted with a membership signup request from a member terminal (step S210), the web server 100 may receive the detailed information of the member, including genome information, as input from the member terminal 200 (step S220).

Then, the web server 100 may store the member's detailed information inputted in step S220 in the member information database 140 (step S230).

The web server 100 can be inputted with a request for joining a premade group, in order to form a social network, from a member terminal 200 (step S240).

Then, the web server 100 may ask the member terminal 200 whether to join a group having members of high genetic similarity to the member (step S241) or join a group having members of high genetic distance to the member (step S243).

If, as a response to the inquiry, a response for joining a group having members of high genetic similarity to the member is received from the member terminal 200, the web server 100 may search for groups formed by members having high genetic similarity to the member (step S251).

However, if, as a response to the inquiry, a response for joining a group having members of high genetic distance to the member is received from the member terminal 200, the web server 100 may search for groups formed by members having high genetic distance to the member (step S253).

The group information searched by the web server 100 may then be provided to the member terminal 200 (step S260). Here, the web server 100 may preferably perform the processing such that the detailed information of these groups is available only if the member terminal 200 has agreed to open the detailed information of its member to the community.

Afterwards, when a request is inputted from the member terminal 200 for joining a searched group, the web server 100 may transmit a signup request message, for requesting signup, to an administrator terminal of the searched group (step S270). Here, the web server 100 may preferably provide the detailed information of the member requesting signup together with the signup request message.

Afterwards, if the web server 100 receives group signup authorization from the administrator terminal, the web server 100 may perform the processing necessary to join the member to the group and form a social network.

As set forth above, certain aspects of the invention can be utilized to satisfy the various personal demands of members by analyzing the gene information of members to form a database of the members' genome information and forming a social network online according to the degree of genetic similarity or distance of the genome information.

Also, certain aspects of the invention can be utilized to form a social network between members having genetic similarity, thus enabling members to use the network immediately when needed (for example, when in need of a transplant, etc.).

In the foregoing, the invention is illustrated and described with reference to certain preferred embodiments. However, the invention is not to be limited to the embodiments disclosed above, and it will be appreciated that those of ordinary skill in the art can implement various modifications without departing from the spirit of the invention. Thus, the scope of the invention is not to be limited by the particular embodiments described herein but is to be defined by the appended claims.

## Claims

1. A system for forming an online social network using genome information, the system comprising:

   a web server configured to receive detailed information of a member, including genome information of the member, from a member terminal as input and create a database of the detailed information, the web server configured to generate a social network on the world wide web based on the genome information of the member.

2. The system of claim 1, wherein the web server comprises:

   a membership signup unit configured to receive a membership signup request as input from a member terminal;

a member information input unit configured to receive detailed information of a member, including the genome information of the member, as input from the member terminal from which the membership signup request is inputted;

a member information database configured to have the detailed information of the member stored therein; and

a social network generator unit configured to form a social network on the world wide web by way of a particular group, based on the genome information of members stored in the member information database, when a request for generating a social network is received as input from the member terminal.

3. The system of claim 2, wherein the social network generator unit comprises:

a group generation processing unit configured to ask whether to generate a group of members having high genetic similarity to a member or a group of members having high genetic distance to the member and generate a group based on a response, when a group generation request for generating a group is received as input from a member terminal; and

a group signup processing unit configured to ask whether to join a group of members having high genetic similarity to a member or a group of members having high genetic distance to the member and join the member to a group based on a response, when a request for joining a pregenerated group is received as input from a member terminal.

4. The system of claim 3, wherein the group generation processing unit, upon receiving a response from a member terminal for generating a group of members having high genetic similarity to the member, accesses the genome information of members stored in the member information database, extracts members having high genetic similarity to the genome information of the member, transmits a request message for inviting signup to a group to terminals of the extracted members, and generates a particular group by grouping the member wishing to generate a group with members accepting the request message.

5. The system of claim 3, wherein the group generation processing unit, upon receiving a response from a member terminal for generating a group of members having high genetic distance to the member, accesses the genome information of members stored in the member information database, extracts members having high genetic distance to the genome information of the member, transmits a request message for inviting signup to a group to terminals of the extracted members, and generates a particular group by grouping the member wishing to generate a group with members accepting the request message.

6. The system of claim 3, wherein the group signup processing unit, upon receiving a response from a member terminal for joining a group of members having high genetic similarity to the member, searches for groups formed by members having high genetic similarity to the member, transmits a signup request message for requesting signup to terminals of administrators of the searched groups, and if group signup authorization is received from a group administrator terminal, joins the member to the group.

7. The system of claim 3, wherein the group signup processing unit, upon receiving a response from a member terminal for joining a group of members having high genetic distance to the member, searches for groups formed by members having high genetic distance to the member, transmits a signup request message for requesting signup to terminals of administrators of the searched groups, and if group signup authorization is received from a group administrator terminal, joins the member to the group.

8. A method for forming an online social network using genome information, the method comprising:

(A) receiving by a web server of detailed information of a member as input from a member terminal, the detailed information including genome information;

(B) storing by the web server of the detailed information of the member inputted in said step (A) in a member information database;

(C) receiving by the web server of a request for generating a social network on the world wide web as input from a member terminal; and

(D) forming by the web server of a social network on the world wide web by way of a particular group, based on the genome information of members stored in the member information database.

9. The method of claim 8, wherein said step (C) comprises:

(C1) receiving by the web server of a group generation request for generating a group as input from the member terminal; and

(C2) asking by the web server of whether to generate a group of members having high genetic similarity to the member or a group of members having high genetic distance to the member.

**10.** The method of claim 9, wherein said step (D) comprises:

(D1) accessing by the web server of the genome information of members stored in the member information database, if as a result of said step (C2) a response is received from the member terminal for generating a group of members having high genetic similarity to the member;

(D2) extracting by the web server of members having high genetic similarity to the genome information of the member and transmitting a request message for inviting signup to a group to terminals of the extracted members; and

(D3) generating by the web server of a particular group by grouping the member wishing to generate a group with members accepting the request message of said step (D2).

**11.** The method of claim 9, wherein said step (D) comprises:

(D1') accessing by the web server of the genome information of members stored in the member information database, if as a result of said step (C2) a response is received from the member terminal for generating a group of members having high genetic distance to the member;

(D2') extracting by the web server of members having high genetic distance to the genome information of the member and transmitting a request message for inviting signup to a group to terminals of the extracted members; and

(D3') generating by the web server of a particular group by grouping the member wishing to generate a group with members accepting the request message of said step (D2').

**12.** The method of claim 8, wherein said step (C) comprises:

(C1) receiving by the web server of a request for joining a pre-generated group as input from the member terminal; and

(C2) asking by the web server of whether to join a group of members having high genetic similarity to the member or a group of members having high genetic distance to the member.

**13.** The method of claim 12, wherein said step (D) comprises:

(D4) searching by the web server for groups formed by members having high genetic similarity to the member, if as a result of said step (C4) a response is received from the member terminal for joining a group formed by members having high genetic similarity to the member;

(D5) transmitting by the web server of a signup request message for requesting signup to terminals of administrators of the groups searched in said step (D4); and

(D6) joining by the web server of the member to a group, if group signup authorization is received from a group administrator terminal.

**14.** The method of claim 12, wherein said step (D) comprises:

(D4') searching by the web server for groups formed by members having high genetic distance to the member, if as a result of said step (C4) a response is received from the member terminal for joining a group formed by members having high genetic distance to the member;

(D5') transmitting by the web server of a signup request message for requesting signup to terminals of administrators of the groups searched in said step (D4'); and

(D6') joining by the web server of the member to a group, if group signup authorization is received from a group administrator terminal.

Fig. 1

Fig. 2

Fig. 3

```
                                      ┌──────────┐
                                      │  start   │
                                      └────┬─────┘
                                           │
                            ┌──────────────▼──────────────┐
                            │   request membership signup  │────── S100
                            └──────────────┬──────────────┘
                                           │
                            ┌──────────────▼──────────────┐
                            │   input member information   │
                            │   including member's genome  │────── S110
                            │         information          │
                            └──────────────┬──────────────┘
                                           │
                            ┌──────────────▼──────────────┐
                            │   store member information   │────── S120
                            │         in database          │
                            └──────────────┬──────────────┘
                                           │                            S130
                                ┌──────────▼────────────┐  no
                                │ does member request    ├──────┐
                                │ search on information  │      │
                                │ of another member?     │      │
                                └──────────┬─────────────┘      │
                                           │ yes                │
  S133                                     │                    │
        ┌──────────────────────┐  no  ┌────▼──────────────────┐ │  S131
   no ──┤ requesting search of  ◄──────┤ requesting search of  │◄─── 
        │ members having high   │      │ members having high   │ │
        │ genetic distance?     │      │ genetic similarity?   │ │
        └──────────┬───────────┘      └───────────┬───────────┘ │
                   │ yes                          │ yes         │
        ┌──────────▼───────────┐      ┌───────────▼───────────┐ │
        │ extract members having│      │ extract members having│ │ S141
        │ high genetic distance │      │ high genetic similarity│
        │ to member             │      │ to member             │
        └──────────┬───────────┘      └───────────┬───────────┘ │
   S143            └──────────────┬───────────────┘             │
                                  │                             │
                       ┌──────────▼──────────────┐              │
                       │ provide extracted member │────── S150   │
                       │      information         │              │
                       └──────────┬──────────────┘              │
                                  │                             │
                       ┌──────────▼──────────────┐  no          │
                       │ requesting group         ├─────────────┤
                       │ generation?              │   S160       │
                       └──────────┬──────────────┘              │
                                  │ yes                         │
                       ┌──────────▼──────────────┐              │
                       │ generate group for member│────── S170   │
                       └──────────┬──────────────┘              │
                                  │◄─────────────────────────────┘
                            ┌─────▼─────┐
                            │    end    │
                            └───────────┘
```

Fig. 4

```
                              ┌─────────┐
                              │  start  │
                              └─────────┘
                                   │
                                   ▼
                    ┌──────────────────────────┐
                    │  request membership signup│────── S210
                    └──────────────────────────┘
                                   │
                                   ▼
                    ┌──────────────────────────┐
                    │   input member information│
                    │   including member's genome│──── S220
                    │        information        │
                    └──────────────────────────┘
                                   │
                                   ▼
                    ┌──────────────────────────┐
                    │   store member information│──── S230
                    │       in database        │
                    └──────────────────────────┘
                                   │
                                   ▼
                                                        S240
                    ╱──────────────────────────╲
                    ╲    does member wish to     ╱ no
                    ╱       join a group?        ╲
                    ╲──────────────────────────╱
                                │ yes
                                ▼
S243
 ╱────────────────────╲  no  ╱────────────────────╲──── S241
 ╲ requesting search of ╱◄────╲ requesting search of ╱
 ╱ members having high  ╲ no  ╱ members having high  ╲
 ╲ genetic distance?    ╱     ╲ genetic similarity?  ╱
 ╲────────────────────╱       ╲────────────────────╱
         │ yes                          │ yes
         ▼                              ▼
 ┌──────────────────┐        ┌──────────────────┐
 │ extract members  │        │ extract members  │──── S251
 │ having high      │        │ having high      │
 │ genetic distance │        │ genetic similarity│
 │ to member        │        │ to member        │
 └──────────────────┘        └──────────────────┘
S253
                 │              │
                 └──────┬───────┘
                        ▼
          ┌──────────────────────────────┐
          │ provide extracted member info │──── S260
          └──────────────────────────────┘
                        │
                        ▼
          ┌──────────────────────────────┐
          │      request group signup     │──── S270
          └──────────────────────────────┘
                        │
                        ▼
          ╱──────────────────────────────╲ no
          ╲ has group signup been authorized? ╱
          ╱──────────────────────────────╲
                        │ yes              S280
                        ▼
          ┌──────────────────────────────┐
          │      join member to group     │──── S290
          └──────────────────────────────┘
                        │
                        ▼
                   ┌─────────┐
                   │   end   │
                   └─────────┘
```

Fig. 5

| Group Info. |
| --- |

49 — Group A

Member A1:
Variation 1: AC
Variation 2: AA
Variation 3: CG
...
Variation M: ij

Member A2
Member A3
...
Member AN

53

Query sample Q:
Variation 1: AC
Variation 1: AA
Variation 1: CG
...
Variation K: ij

50 — Group B

51 — Group ...

52 — Group M

If there are m online social groups (A..M) with n members (I...N), each member has k genetic loci (1..K), and a locus K has two alleles, a query sample Q's individual distance to a member N of group A ($Q_{AN}$) will be calculated as follows:

1) The proportion ($P_{AN}$) of shared alleles between individuals will be estimated by
- $P_{AN} = \sum_1{}^k S/2k$
- Shared allele S on each locus is counted according to submitted variation types ij; 1) If Nth alleles between the query and a group member are AA:AA, S will be counted as "2", 2) If alleles are AT:AA, S will be "1", and 3) If alleles are AA:TT, S will be "0".

2) The individual distance $Q_{AN}$ will be calculated as follows:
- $Q_{AN} = 1 - P_{AN}$
A sample Q's group similarity ($GS_A$) and group distance ($GD_A$) to group A will be

3) $GS_A = \sum P_{AN}/n$
4) $GD_A = \sum Q_{AN}/n$

System will return group names according to the size of GS and GD

5) Order of group similarity: $GS_B > GS_A > GS_C > ... > GS_M$
6) Order of group distance: $GD_B > GD_A > GD_C > ... > GD_M$

14

**Europäisches Patentamt**
**European Patent Office**
**Office européen des brevets**

## EUROPEAN SEARCH REPORT

Application Number

EP 11 15 8611

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/017520 A1 (NAVIGENICS INC [US]; MOORE STEPHEN M [US]; NIERENBERG MICHAEL A [US];) 11 February 2010 (2010-02-11) * paragraphs [0146] - [0152] * ----- | 1-14 | INV. G06Q10/00 G06Q50/00 |
| X | GB 2 444 410 A (NAVIGENICS INC [US]; FILIPPONE MELISSA FLOREN [US]; CARGILL MICHELE [U) 4 June 2008 (2008-06-04) * paragraphs [0035] - [0052], [0063], [0065] * ----- | 1-14 | |

TECHNICAL FIELDS
SEARCHED (IPC)

G06Q

The present search report has been drawn up for all claims

| Place of search | Date of completion of the search | Examiner |
|---|---|---|
| The Hague | 1 September 2011 | Lutz, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 397 978 A1**

## ANNEX TO THE EUROPEAN SEARCH REPORT
## ON EUROPEAN PATENT APPLICATION NO.

EP 11 15 8611

This annex lists the patent family members relating to the patent documents cited in the above-mentioned European search report.
The members are as contained in the European Patent Office EDP file on
The European Patent Office is in no way liable for these particulars which are merely given for the purpose of information.

01-09-2011

| Patent document cited in search report | Publication date | Patent family member(s) | Publication date |
|---|---|---|---|
| WO 2010017520 A1 | 11-02-2010 | AU 2009279434 A1<br>CN 102171697 A<br>EP 2321753 A1<br>GB 2478065 A<br>KR 20110053995 A<br>US 2010042438 A1 | 11-02-2010<br>31-08-2011<br>18-05-2011<br>24-08-2011<br>24-05-2011<br>18-02-2010 |
| GB 2444410 A | 04-06-2008 | AU 2007325021 A1<br>CA 2671267 A1<br>EP 2102651 A2<br>JP 2010522537 A<br>WO 2008067551 A2 | 05-06-2008<br>05-06-2008<br>23-09-2009<br>08-07-2010<br>05-06-2008 |

EPO FORM P0459

For more details about this annex : see Official Journal of the European Patent Office, No. 12/82